# EUROPEAN PATENT SPECIFICATION

(11) **EP 3 076 157 B1**
(45) Date of publication and mention of the grant of the patent: **15.04.2020**
(21) Application number: 16162321.0
(22) Date of filing: 24.03.2016
(51) Int. Cl.: G01N 15/12, G01N 35/10, G01N 15/14, G01N 15/10, G01N 15/00

(54) **BLOOD MEASURING DEVICE AND BLOOD MEASURING DEVICE CONTROL METHOD**
BLUTMESSVORRICHTUNG UND BLUTMESSVORRICHTUNGSSTEUERUNGSVERFAHREN
DISPOSITIF DE MESURE DE SANG ET PROCÉDÉ DE COMMANDE ASSOCIÉ

(30) Priority: 27.03.2015 JP 2015066853
(43) Date of publication of application: 05.10.2016
(73) Proprietor: SYSMEX CORPORATION, Kobe-shi, Hyogo 651-0073 (JP)
(72) Inventor: Nagai, Takaaki, Hyogo, 651-0073 (JP); Toyoda, Akio, Hyogo, 651-0073 (JP)
(74) Representative: Hoffmann Eitle

(56) References cited:
- EP-A2- 2 224 247
- WO-A1-98/02727
- WO-A2-98/21594

## Description

### FIELD OF THE INVENTION

The invention relates to a blood measuring device and blood measuring device control method.

### BACKGROUND

Blood measuring devices which automatically measure samples of blood and the like are known. Blood measuring devices prepare measurement samples to be measured by diluting the sample using a sample diluting liquid. Japanese Patent Application Publication No. 2010-197292 discloses a sample preparing device which dilutes a high concentration reagent with RO (reverse osmosis) water produced through an RO membrane treatment, and uses the prepared low concentration reagent as a sample diluting liquid.

EP 2 224 247 A2 discloses a reagent preparing device, specimen measuring device and reagent preparing method. The reagent preparing device comprises a first liquid storage unit and controls a first liquid discarding unit that discards the first liquid from the first liquid storage unit when an accumulated time reaches a predetermined time.

WO 98/21594 A2 discloses an automatic chemistry analyzer for high volume chemical analyses. It has a heated reaction cup. On the side, it discloses inexpensive application of deionized water in a rinsing step.

WO 98/02727 A1 discloses automated methods for distinguishing and differentiating cells in a blood sample. The instrument is configured to perform multiple tests to be run on a blood sample in one single device.

The blood measuring device disclosed in Japanese Patent Application Publication No. 2010-197292 uses the sample diluting liquid as a sheath fluid for flowing the measurement sample to a flow cytometer and as a washing liquid for washing the sample preparing part and the detection parts of the blood measuring device. In this case, it may become impossible to measure a sample or smooth measurement may become inhibited when the high concentration reagent used to prepare the sample diluting liquid becomes depleted due to having used a large quantity of sample diluting liquid. Therefore, there is demand to inhibit the consumption of the high concentration reagent.

### SUMMARY OF THE INVENTION

This object is accomplished by the subject matter of the independent claims. The dependent claims concern particular embodiments.

The scope of the present invention is defined solely by the appended claims, and is not affected to any degree by the statements within this summary.

According to the invention, it is possible to inhibit the consumption of high concentration reagent by the blood measuring device.

### BRIEF DESCRIPTION OF THE DRAWINGS

FIG. 1 is a block diagram showing the structure of the blood measuring device of the embodiment;
FIG. 2(a) shows the aspirating tube and washing device of the embodiment viewed from the X-axis negative direction, and FIG. 2(b) shows the washing device of the embodiment viewed from the Z-axis positive direction;
FIG. 3(a) shows the structure of the flow cell of the electrical resistance detecting part of the embodiment, FIG. 3(b) shows the structure of the flow cell of the optical detecting part of the embodiment, FIG. 3(c) shows the structure of the hemoglobin measuring part of the embodiment, and FIG. 3(d) shows the structure of the float switch of the fluid supplying part of the embodiment;
FIG. 4(a) and (b) show the structure of the fluid supplying part of the embodiment;
FIG. 5 shows the structure of the fluid supplying part of the embodiment; FIG. 6(a) through (c) respectively show the controls performed by the controller in the first measurement, second measurement, and third measurement of the embodiment;
FIG. 7A and 7B are flow charts respectively showing the controls performed by the controller in the first sample preparing part and the second sample preparing part of the embodiment;
FIG. 8A through 8C are flow charts respectively showing the controls performed by the controller in washing the electrical resistance detecting part, optical resistance detecting part, and hemoglobin measuring part of the embodiment; and
FIG. 9A through 9C are flow charts showing the controls performed by the controller in washing the aspirating tube, the shutdown process, and the anomaly process of the pure water reservoir.

### DESCRIPTION OF THE EMBODIMENTS OF THE INVENTION

The first through third embodiments described below apply the present invention in an apparatus which performs examination and analysis of blood by detecting the red blood cells and the like contained in a blood sample, and counting each blood cell.

The embodiment described below applies the invention to a device which measures blood cells and the like contained in blood samples. Note that the pure water used in the invention is water subjected to treatment to eliminate impurities and includes RO water, purified water, deionized water, and distilled water. The purity of the pure water is not limited.

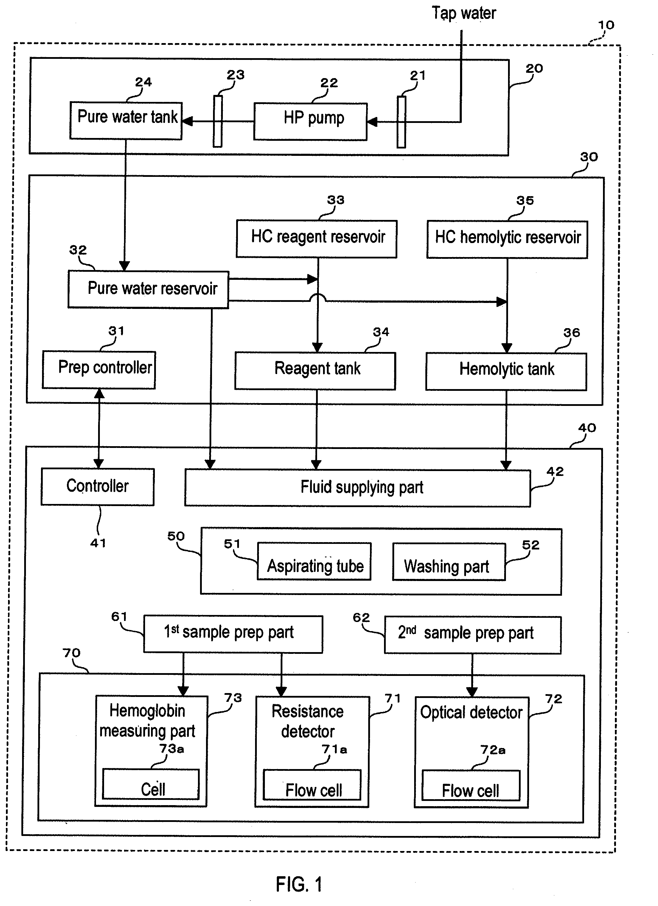

As shown in FIG. 1, the blood measuring device 10 is provided with a pure water supply unit 20, reagent preparation unit 30, and measurement unit 40. The pure water supply unit 20, reagent preparation unit 30, and measurement unit 40 also may be configured as respectively separate devices. That is, the pure water supply unit 20 also may be configured as a pure water supply device, the reagent preparation unit 30 also may be configured as a reagent preparation device, and the measurement unit 40 also may be configured as a measurement device.

The pure water supply unit 20 has a filter 21, high pressure pump 22, membrane 23, and pure water tank 24. The filter 21 removes impurities contained in tap water. The high pressure pump 22 applies high pressure to the tap water passing through the filter 21 to transport the water molecules through the membrane 23. The pure water tank 24 stores the pure water that has been transported through the membrane 23. As shall be described below, the pure water also may be used in measurements and washing of the measurement unit 40 in addition to diluting high concentration reagent and high concentration hemolytic agent.

RO water is used as the pure water in the present embodiment. RO water is water from which impurities have been removed by passing through reverse osmosis membrane, that is, an RO (reverse osmosis) membrane. The membrane 23 of the embodiment is therefore an RO membrane.

The reagent preparation unit 30 has a preparation controller 31, pure water reservoir 32, high concentration reagent reservoir 33, reagent tank 34, high concentration hemolytic agent reservoir 35, and hemolytic agent tank 36. Preparation controller 31 is, for example, a CPU. Fluid is transported within the reagent preparation unit 30 by the preparation controller 31 controlling the operation of an air pressure source and valves (not shown in the drawings) of the reagent preparation unit 30.

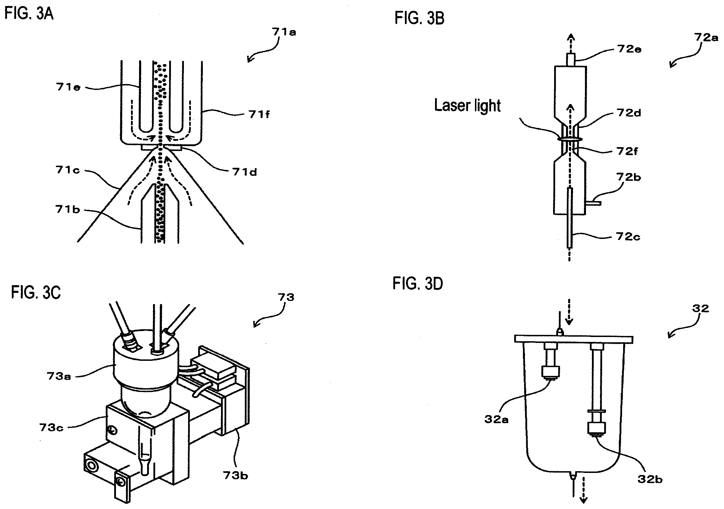

The pure water reservoir 32 stores pure water. Referring to FIG. 3D, the pure water reservoir 32 has float switches 32a and 32b, which are described below. The preparation controller 31 supplies the pure water from the pure water tank 24 to the pure water reservoir 32 so that the stored quantity in the pure water reservoir 32 is within a predetermined range based on the detection signals of the float switches 32a and 32b.

The high concentration reagent reservoir 33 stores high concentration reagent received from the high concentration reagent tank 34. The high concentration reagent includes conductive electrolyte and antiseptic. The electrolyte of the embodiment is NaCl. (2-pyridylthio-1-oxide) sodium is used as the antiseptic. The antiseptic, for example, may be configured with TKM-A (made by API Corporation, Ltd.) as a material containing (2-pyridylthio-1-oxide) sodium. The reagent tank 34 stores low concentration reagent prepared by mixing the high concentration reagent held in the high concentration reagent reservoir 33 and the pure water held in the pure water reservoir 32. The low concentration reagent is prepared by supplying a predetermined amount of pure water and a predetermined amount of high concentration reagent to the reagent tank 34. The low concentration reagent is set to osmotic pressure so as to not substantially affect the blood cells in the blood sample.

The high concentration hemolytic agent reservoir 35 stores high concentration hemolytic agent. The hemolytic agent tank 36 stores hemolytic agent prepared by mixing the high concentration hemolytic agent held in the high concentration hemolytic agent reservoir 35 and the pure water held in the pure water reservoir 32. The hemolytic agent is prepared by supplying a predetermined amount of pure water and a predetermined amount of high concentration hemolytic agent to the hemolytic agent tank 36.

The measurement unit 40 has a controller 41, fluid supplying part 42, aspirating part 50, first sample preparing part 61, second measurement preparing part 62, and measuring part 70. The aspirating part 50 has an aspirating tube 51 and a washing part 52. The measuring part 70 has a resistance detector 71, optical detector 72, and hemoglobin measuring part 73.

Note that although the controller 41 is incorporated within the measurement unit 40 in the present embodiment, the controller 41 also may be a separate personal computer or the like external to the measurement unit 40.

Controller 41 is, for example, a CPU. The controller 41receives signals output by each part of the measurement unit 40, and controls the operations of each part of the measurement unit 40. The controller 41 communicates with the preparation controller 31.

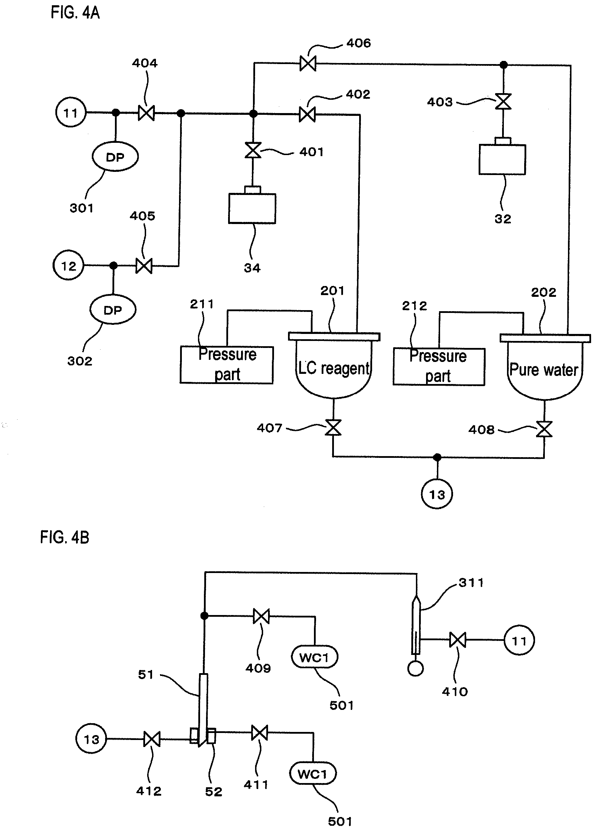

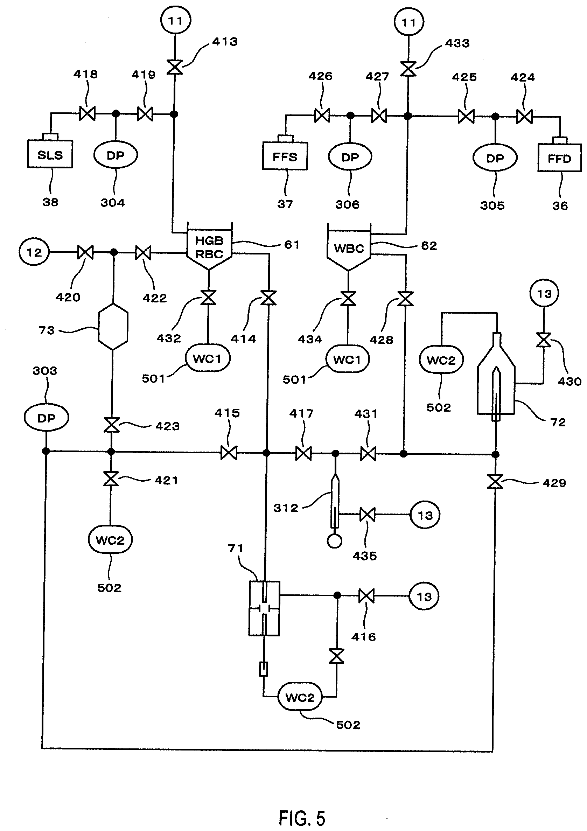

The fluid supplying part 42 is connected to the pure water reservoir 32, reagent tank 34, and hemolytic agent tank 36. The fluid supplying part 42 supplies pure water and low concentration reagent to the aspirating tube 50, supplies low concentration reagent to the first sample preparing part 61, supplies pure water, low concentration reagent, and hemolytic agent to the second sample preparing part 62, and supplies pure water and low concentration reagent to the measuring part 70. The structure of the fluid supplying part 42 is described below referring to FIG. 4A, 4B, and FIG. 5.

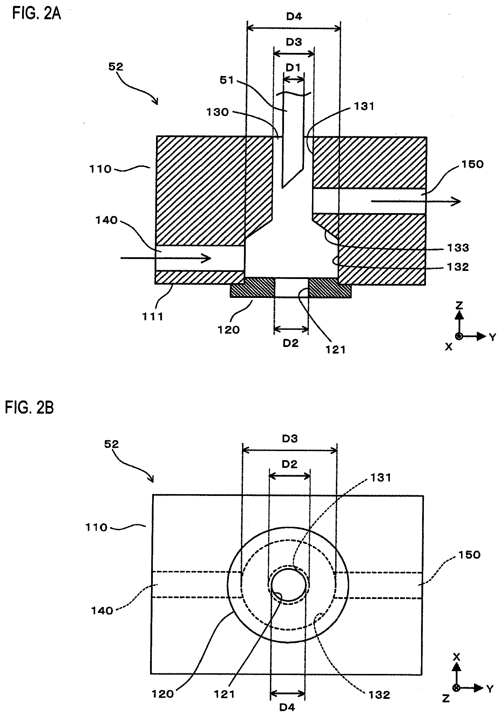

The aspirating tube 51 aspirates the blood sample from the test tube received by the blood measuring device 10, and discharges the blood sample to the first sample preparing part 61 and the second sample preparing part 62. The washing part 52 washes the exterior of the aspirating tube 51. The structure of the washing part 52 is described below referring to FIG. 2A and 2B.

The first sample preparing part 61 prepares a first measurement sample by mixing the blood sample and the low concentration reagent. The second sample preparing part prepares a second measurement sample by mixing the blood sample, hemolytic agent, and stain. The hemolytic agent lyses red blood cells. The stain stains the nucleic acid of white blood cells. The stain is supplied from the stain reservoir 37 which is described later referring to FIG. 5. The first sample preparing part 61 prepares a third measurement sample by mixing the first measurement sample and hemoglobin hemolytic agent. The hemoglobin hemolytic agent transforms the hemoglobin in the blood to SLS-hemoglobin. The hemoglobin hemolytic agent is supplied from a hemoglobin hemolytic agent tank 3 which is described later referring to FIG. 5. 8

The measuring part 70 measures the first through third measurement samples. Specifically, the resistance detector 71 measures the first measurement sample, that is, performs a first measurement, by a sheath flow detection method. The resistance detector 71 has a flow cell 71a, and measures the first measurement sample flowing with the sheath fluid through the flow cell 71a. The first measurement is a measurement related to red blood cells and platelets. The optical detector 72 measures the second measurement sample, that is, performs a second measurement by a flow cytometric method. The optical detector 72 has a flow cell 72a, and measures the second measurement sample flowing with the sheath fluid through the flow cell 72a. The second measurement is a measurement related to white blood cells. The hemoglobin measuring part 73 measures the third measurement sample, that is, performs a third measurement, by a SLS-hemoglobin method. The hemoglobin measuring part 73 has a cell 73a, and measures the third measurement sample in the cell 73a. The third measurement is a measurement related to hemoglobin.

The controls during measurements are summarized below.

In the first measurement, the fluid supplying part 42 flows the first measurement sample to the flow cell 71a while supplying low concentration reagent as a sheath fluid to the flow cell 71a of the resistance detector 71 based on the controls of the controller 41. The resistance detector 71 measures the red blood cells and platelets in the first measurement sample flowing through the flow cell 71a, and obtains detection signals based on each blood cell. The detection signals of the resistance detector 71 are processed in a signal processing circuit (not shown in the drawing) and transmitted to the controller 41. The structure of the flow cell 71a is described later referring to FIG. 3A.

In the second measurement, the fluid supplying part 42 flows the second measurement sample to the flow cell 72a while supplying pure water as a sheath fluid to the flow cell 72a of the optical detector 72 based on the controls of the controller 41. The optical detector 72 measures the white blood cells in the second measurement sample flowing through the flow cell 72a, and obtains detection signals based on each blood cell. The detection signals of the optical detector 72 are processed in a signal processing circuit (not shown in the drawing) and transmitted to the controller 41. The structure of the flow cell 72a is described later referring to FIG. 3B.

In the third measurement, the fluid supplying part 42 supplies pure water to the cell 73a of the hemoglobin measuring part 73, then deposits the third measurement sample in the cell 73a based on the controls of the controller 41. The hemoglobin measuring part 73 obtains detection signals based on the light absorption by the third measurement sample. The detection signals of the hemoglobin measuring part 73 are processed in a signal processing circuit (not shown in the drawing) and transmitted to the controller 41. The structure of the hemoglobin measuring part 73 is described later referring to FIG. 3C.

The controls during washing are summarized below.

When a washing operation is performed by the measurement unit 40, the fluid supplying part 42 washes the sites least affecting the measurement results of the first through third measurement samples using pure water, and washes the sites affecting the measurement results of the first through third measurement samples using low concentration reagent based on the controls of the controller 41. Specifically, the washing operation occurs as follows.

The fluid supplying part 42 washes the interior of the aspirating tube 51 using low concentration reagent, and washes the exterior of the aspirating tube 51 using pure water via the washing part 52 based on the controls of the controller 41. The fluid supplying part 42 washes the first sample preparing part 61 using low concentration reagent based on the control of the controller 41. The fluid supplying part 42 washes the second sample preparing part 62 using pure water based on the control of the controller 41. The fluid supplying part 42 washes the resistance detector 71 using low concentration reagent based on the control of the controller 41. The fluid supplying part 42 washes the optical detector 72 using pure water based on the control of the controller 41.

When the controller 41 receives a shutdown instruction of the measurement unit 40, the fluid supplying part 42 washes the predetermined site of the measurement unit 40, which was previously washed with pure water, using low concentration reagent based on the controls of the controller 41. Specifically, the predetermined sites are the second sample preparing part 62, optical detector 72, hemoglobin measuring part 73, and exterior of the aspirating tube 51.

As shown in FIG. 2A and 2B, the washing part 52 is arranged at a position corresponding to the aspirating tube 51. The washing part 52 is supported at a predetermined position in the vertical direction, that is, supported so as to be movable in the horizontal direction together with the aspirating tube 51. FIG. 2A and (b) show the X, Y, and Z axes forming mutually 90 degree angles for convenience. The Z axis positive direction is the vertically upward direction.

The washing part 52 has a body part 110 and a diaphragm part 120. A through-hole 130, supply path 140, and discharge path 150 are formed in the body part 110. The diaphragm part 120 is arranged in the center of the bottom surface of the body part 110. A through-hole 121 which passes through the diaphragm part 120 in the vertical direction is formed in the diaphragm part 120. The inner diameter D2 of the through-hole 121 is greater than the outer diameter D1 of the aspirating tube 51, but less than the inner diameter D3 of a top hole 131 which is described later. The cross section of the horizontal plane of the through-hole 121 is circular. The center axis of the diaphragm part 120 and the center axis of the through-hole 130 match.

The through-hole 130 passes through the body part 110 in the vertical direction, and includes the top hole 131 and the bottom hole 132. The top hole 131 and the bottom hole 132 are respectively positioned at the top and the bottom of the through-hole 130. The cross section of the horizontal plane of the top hole 131 and the bottom hole 132 is invariably circular. The internal diameter D3 of the top hole 131 is less than the inner diameter D4 of the bottom hole 132. A tapered portion 133 is provided between the top hole 131 and the bottom hole 132 so that the inner diameter gradually increases from the top hole 131 toward the bottom hole 132. The supply path 140 is formed to extend in a horizontal direction at a position corresponding to the bottom hole 132. The discharge path 150 is formed to extend in a horizontal direction at a position corresponding to the top hole 131.

The bottom end of the aspirating tube 51 is needle shaped and is capable of penetrating the sealed lid of the test tube. The aspirating tube 51 is moved downward and penetrates the sealed lid of the test tube to aspirate the blood sample. In this state, the aspirating tube 51 aspirates the blood sample. When the aspiration of the blood sample is completed, the aspirating tube 51 is moved upward, then moved horizontally until above the first sample preparing part 61 or the second sample preparing part 62. The aspirating tube 51 is moved downward and discharges the blood sample into the first sample preparing part 61 or the second sample preparing part 62 when discharging the blood sample. Thereafter, the aspirating tube 51 is raised.

The aspirating tube 51 is moved vertically through the through-hole 130 and the diaphragm part 120 when aspirating or discharging the blood sample. The washing part 52 washes the exterior of the aspirating tube 51 when the aspirating tube 51 is moved in the upward direction. Specifically, pure water supplied from the supply path 140 into the through-hole 130 rises while swirling in the bottom hole 132, and is discharged from the discharge path 150 positioned at the top hole 131. The washing part 52 washes the exterior of the aspirating tube 51 which ascends within the through-hole 130 using the swirling flow.

As shown in FIG. 3A, the flow cell 71a of the resistance detector 71has a sample nozzle 71b, chamber 71c, aperture 71d, collection tube 71e, and chamber 71f.

The sample nozzle 71bsends the first measurement sample upward. The chamber 71c has a tapered shape which narrows in the upward direction. Low concentration reagent is supplied as sheath fluid into the chamber 71c. The first measurement sample moves through the aperture 71d to the collection tube 71e while circumscribed in sheath fluid. The blood cells contained in the first measurement sample pass through the aperture 71d aligned in a row.

An electrode is provided in the aperture 71d. In the first measurement, a DC electrical current is supplied between the electrodes of the aperture 71d, and the change in current resistance is detected when the first measurement sample passes through the aperture 71d. As described above, the first measurement sample is prepared by mixing the blood sample and low concentration reagent, and the low concentration reagent is conductive because it contains electrolytes. Since the DC resistance increases when the blood cells of the first measurement sample pass through the aperture 71d, the detection signal therefore reflects information of the blood cell passing through the aperture 71d. Accordingly, red blood cells and platelets can be counted by the detection signals. The resistance detector 71 outputs the detection signals to a later stage signal processing circuit.

The chamber 71f is supplied low concentration reagent as a sheath fluid on the collection tube 71e side. The sheath fluid flows downward toward the outer region of the collection tube 71e of the chamber 71f. The sheath fluid flowing outside the collection tube 71e arrives at the bottom end of the chamber 71f, then flows inside the collection tube 71e. Thus, a backflow of the blood cells that have passed through the aperture 71d is prevented, which prevents blood cell detection error.

When washing the resistance detector 71, low concentration reagent is supplied to the sample nozzle 71b, and the low concentration reagent is sent upward from the sample nozzle 71b. Low concentration reagent is supplied to the chambers 71c and 71f similar to the case of the first measurement. Washing of the resistance detector 71 is performed in this way.

As shown in FIG. 3B, the flow cell 72a of the optical detector 72 is provided with a sheath fluid supply port 72b, sample nozzle 72c, pore part 72d, and drain port 72e.

The sheath fluid supply port 72b supplies pure water as sheath fluid into the flow cell 72a. The sample nozzle 72c sends the second measurement sample upward into the flow cell 72a. The second measurement sample progresses through a flow path 72f formed in the pore part 72d while encapsulated in the sheath fluid, and toward the drain port 72e. The blood cells contained in the second measurement sample pass through the aperture 72f aligned in a row.

Laser light of a predetermined wavelength irradiates the flow path 72f. In the second measurement, when the second measurement sample passing through the flow path 72f is irradiated by laser light, forward scattered light, side scattered light, and fluorescent light are produced by the blood cells in the second measurement sample. The intensity of the forward scattered light, side scattered light, and fluorescent light is detected by the light receiving part of the optical detector 72. The intensity of the forward scattered light reflects information related to the size of the blood cell, the intensity of the side scattered light reflects information related to the interior of the blood cell, and the fluorescent light reflects information related to the degree of staining of the blood cell. Accordingly, the number of white blood cells can be determined by the detection signals. The optical detector 72 outputs the detection signals to a later stage signal processing circuit.

When washing the optical detector 72, pure water is supplied to the sample nozzle 72c and expelled upward from the sample nozzle 72c. Pure water is supplied to the sheath fluid supply port 72b similar to the case of the second measurement. Washing of the optical detector 72 is performed in this way.

As shown in FIG. 3C, the hemoglobin measuring part 73 is provided with a cell 73a, light emitting diode 73b, and light receiving element 73c.

The cell 73a is made of a high light transmitting plastic material. The light emitting diode 73b irradiates the cell 73a with light of a wavelength having a high absorption rate by SLS-hemoglobin. The light receiving element 73c is arranged facing the light emitting diode 73b with the cell 73a disposed therebetween so as to receive the light transmitted through the cell 73a.

The cell 73a is filled with pure water beforehand. During the third measurement, the third measurement sample is supplied to the cell 73a and held in the cell 73a. In this state, the light emitting diode 73b irradiates light, and the transmission light is received by the light receiving element 73c. Since the cell 73a is made of a high light transmitting plastic material, the light receiving element 73c receives only the transmission light which is not absorbed by the third measurement sample from among the light emitted from the light emitting diode 73b. The light receiving element 73c detects the intensity of the transmitted light. The detection signal corresponds to the degree of absorbance. The hemoglobin measuring part 73 outputs the detection signals to a later stage signal processing circuit. In the signal processing circuit, this absorbance is compared to the previously measured absorbance of the low concentration reagent alone.

As shown in FIG. 3D, pure water is supplied from the top of the pure water reservoir 32, and pure water is drawn off from the bottom. The pure water reservoir 32 is provided with float switches 32a and 32b. The float switch 32a detects when the upper limit of the storage capacity of the pure water reservoir 32 is attained. The float switch 32b detects when the lower limit of the storage capacity of the pure water reservoir 32 is attained. The preparation controller 31 supplies the pure water of the pure water tank 24 to the pure water reservoir 32 to maintain the pure water at or above the lower limit and at or below the upper limit within the pure water reservoir 32 based on the detection signals of the float switches 32a and 32b.

The controls performed by the controller 41 for the fluid supplying part 42 are described below referring to the structure of the fluid supplying part 42 shown in FIG. 4A, 4B and FIG. 5. In FIG. 4A, 4B and FIG. 5, the positions 11 through 13 indicate positions on the flow path of the fluid supplying part 42. That is, position 11 in FIG. 4A is linked to position 11 in FIG. 4B and FIG. 5. Position 12 in FIG. 4A is linked to position 11 in FIG. 5. Position 13 in FIG. 4A is linked to position 13 in FIG. 4B and FIG. 5.

As shown in FIG. 4A, and 4B and FIG. 5, the fluid supplying part 42 is provided with a reagent chamber 201, pure water chamber 202, pressure parts 211 and 212, diaphragm pumps 301 through 306, syringe pumps 311 and 312, valves 401 through 435, waste chambers 501 and 502.

Pressure parts 211 and 212 apply positive pressure and negative pressure to the flow path. The diaphragm pumps 301 through 306 uptake a predetermined amount of fluid on the flow path side when negative pressure is actuated, and output the uptaken fluid to the flow path side when a positive pressure is actuated. The valves 401 through 435 are configured to open and close electromagnetically. In the following description, valves 401 through 435 are assumed to be closed unless otherwise specified. A pressure part not shown in the drawing is connected to the waste chambers 501 and 502, and fluid is moved to the waste chambers 501 and 502 by this pressure part. The pressure parts 211 and 212, diaphragm pumps 301 through 306, syringe pumps 311 and 312, and valves 401 through 435 are controlled by the controller 41.

Referring to FIG. 4A, the controller 41 transports the low concentration reagent of the reagent tank 34 to the chamber 201 by opening valves 401 and 402 and actuating the pressure part 211. The controller 41 transports the pure water of the pure water reservoir 32 to the chamber 202 by opening valve 403 and actuating the pressure part 212.

The controller 41 transports low concentration reagent of the reagent chamber 201 to position 11 by actuating valves 402 and 404 and the diaphragm pump 301. The controller 41 transports low concentration reagent of the reagent chamber 201 to position 12 by actuating valves 402 and 405 and the diaphragm pump 302. The controller 41 transports the pure water of the pure water tank 202 to position 11 by actuating valves 406 and 404 and the diaphragm pump 301. The controller 41 transports the pure water of the pure water tank 202 to position 12 by actuating valves 406 and 405 and the diaphragm pump 302.

The controller 41 transports low concentration reagent of the reagent chamber 201 to position 13 by actuating valve 407 and the pressure part 211. The controller 41 transports the pure water of the pure water tank 202 to position 13 by actuating valve 408 and the pressure part 212.

In the following description, the operations of respectively transporting pure water and low concentration reagent to positions 11 through 13 are accomplished by the controller 41 which controls the fluid supplying part 42 as described above.

Referring to FIG. 4B, the controller 41 controls the aspirating tube 51 to suction a blood sample by actuating the syringe pump 311. The controller 41 opens the valve 409 when suctioning the blood sample. The controller 41 controls the aspirating tube 51 to discharge the blood sample by actuating the syringe pump 311. The blood sample suctioned from the test tube is supplied to the first sample preparing part 61 and the second sample preparing part 62 in this way.

The controller 41 transports the low concentration reagent which was moved to position 11 from the syringe pump 311 to the flow path connected to aspirating tube 51. The controller 41 transports the low concentration reagent which was moved from the syringe pump 311 to the aspirating tube 51 to the waste chamber 501 by opening valve 411. Specifically, the low concentration reagent discharged from the tip of the aspirating tube 51 is transported to the valve 411 side through the discharge path of the washing part 52 shown in FIG. 2A and 2B. The interior of the aspirating tube 51 is washed in this way.

The controller 41 moves the pure water at position 13 through the washing part 52 to the waste chamber 501 by opening valves 412 and 411. Specifically, the pure water which was moved to the washing part 52 is transported to the valve 411 side through the supply path 140 and discharge path 150 of the washing part 52 shown in FIG. 2A and 2B. The exterior of the aspirating tube 51 is washed in this way.

Referring to FIG. 5, the control of the fluid supplying part 42 by the controller 41 is described below referring to FIG. 5.

The controller 41 supplies only a predetermined amount of the low concentration reagent from position 11 to the first sample preparing part 61 by opening valve 413. The controller 41 discharges a predetermined amount of the blood sample through the aspirating tube 51to the first sample preparing part 61. The controller 41 supplies only a predetermined amount of the low concentration reagent from position 11 to the first sample preparing part 61 by opening valve 413. The first measurement sample is prepared in this way in the first sample preparing part 61.

From the first measurement sample of the first sample preparing part 61, the controller 41 positions a predetermined amount of the first measurement sample in the flow path between the first sample preparing part 61 and the resistance detector 71 by opening valves 414 and 415 and actuating the diaphragm pump 303. The controller 41 supplies the low concentration reagent at position 13 as a sheath fluid to the resistance detector 71 by opening valve 416. The controller 41 supplies the first measurement sample positioned in the flow path between the first sample preparing part 61 and the resistance detector 71 to the resistance detector 71 by opening valve 417 and actuating the syringe pump 312. The collection tube 71e shown in FIG. 3A is connected to the waste chamber 502, and the first measurement sample and low concentration reagent collected in the collection tube 71e are supplied to the waste chamber 502. The first measurement by the resistance detector 71 is performed in this way.

The control of the fluid supplying part 42 by the controller 41 when performing the third measurement is described below.

The controller 41 supplies only a predetermined amount of the hemoglobin hemolytic agent of the hemoglobin hemolytic agent tank 38 to the first sample preparing part 61 by actuating valves 418 and 419 and the diaphragm pump 304. The first sample preparing part 61 prepares a third measurement sample by mixing the remaining first measurement sample and hemoglobin hemolytic agent.

The controller 41 supplies the pure water at position 12 to the hemoglobin measuring part 73 by opening valve 420 when the cell 73a of the hemoglobin measuring part 73 is not filled with pure water. The cell 73a of the hemoglobin measuring part 73 is filled with pure water in this way. The controller 41 transports the fluid taken in by the diaphragm pump 303 to the waste chamber 502 by opening valve 421 and actuating the diaphragm pump 303.

The controller 41 supplies the third measurement sample of the first sample preparing part 61 to the hemoglobin measuring part 73 by actuating valves 422 and 423 and the diaphragm pump 303. Thus, the third measurement sample is supplied to the cell 73a and the third measurement is performed by the hemoglobin measuring part 73. When the third measurement is completed, the controller 41 sends the fluid in the cell 73a to the waste chamber 502 by opening valves 423 and 421. The controller 41 supplies the pure water from position 12 to the hemoglobin measuring part 73 by opening valve 420. Pure water fills the cell 73a for a subsequent third measurement.

The control of the fluid supplying part 42 by the controller 41 when performing the second measurement is described below.

The controller 41 supplies only a predetermined amount of the hemolytic agent of the hemolytic agent tank 36 to the second sample preparing part 62 by actuating valves 424 and 425 and the diaphragm pump 305. The controller 41 discharges only a predetermined amount of the blood sample through the aspirating tube 51 to the second sample preparing part 62. The controller 41 supplies only a predetermined amount of the staining agent of the stain reservoir 37 to the second sample preparing part 62 by actuating valves 426 and 427 and the diaphragm pump 306. The controller 41 supplies only a predetermined amount of the hemolytic agent of the hemolytic agent tank 36 to the second sample preparing part 62 by actuating valves 424 and 425 and the diaphragm pump 305. The second measurement sample is prepared in this way in the second sample preparing part 62.

The controller 41 positions the second measurement sample of the second sample preparing part 62 in the flow path between the second sample preparing part 61 and the optical detector 72 by opening valves 428 and 429 and actuating the diaphragm pump 303. The controller 41 supplies pure water from position 13 as sheath fluid to the optical detector 72 by opening valve 430. The controller 41 supplies the second measurement sample positioned in the flow path between the second sample preparing part 62 and the optical detector 72 to the optical detector 72 by opening valve 431 and actuating the syringe pump 312. The second measurement sample and pure water collected from the drain port 72e shown in FIG. 3B are supplied to the waste chamber 502. The second measurement by the optical detector 72 is performed in this way.

The control of the fluid supplying part 42 by the controller 41 when washing the first sample preparing part 61, second sample preparing part 62, resistance detector 71, and optical detector 72 is described below.

The controller 41 supplies the low concentration reagent from position 11 to the first sample preparing part 61 by opening valve 413. Thereafter, the controller 41 sends the fluid within the first sample preparing part 61 to the waste chamber 501 by opening valve 432. The washing of the first sample preparing part 61 concludes in this way. The controller 41 supplies the pure water from position 11 to the second sample preparing part 62 by opening valve 433. Thereafter, the controller 41 sends the fluid within the second sample preparing part 62 to the waste chamber 501 by opening valve 434. The washing of the second sample preparing part 62 concludes in this way.

The controller 41 supplies the low concentration reagent at position 13 to the resistance detector 71 by opening valves 435 and 417. The controller 41 supplies the low concentration reagent at position 13 to the resistance detector 71 similar to sheath fluid by opening valve 416. Washing of the resistance detector 71 concludes in this way. The controller 41 supplies pure water from position 13 as sheath fluid to the optical detector 72 by opening valves 435 and 431. The controller 41 supplies pure water from position 13 to the optical detector 72 similar to sheath fluid by opening valve 430. Washing of the optical detector 72 concludes in this way.

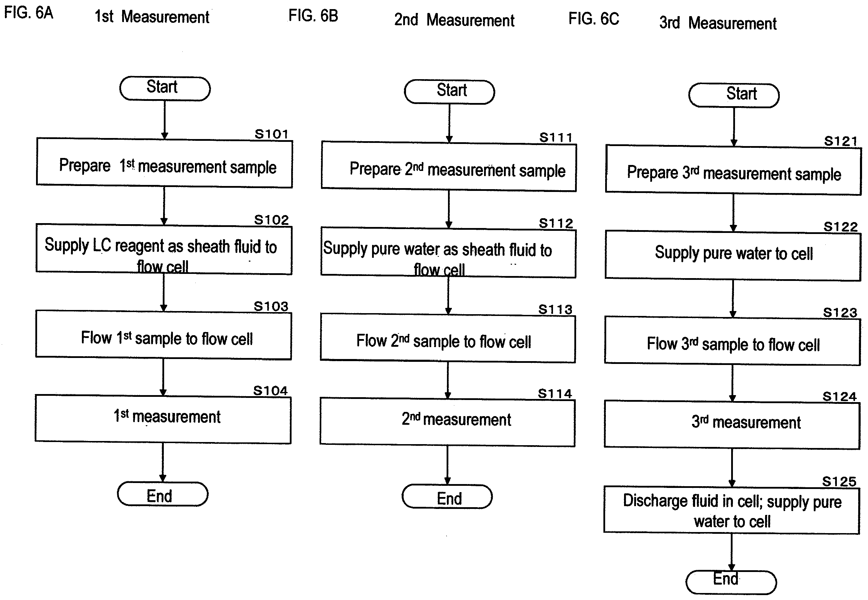

The controls performed by the controller 41 are described below referring to the flow charts shown in FIG. 6A through FIG. 9C. The process in each step in the following flowcharts are performed by the controller 41 controlling each part of the fluid supplying part 42 as described referring to FIG. 4A, 4B and FIG. 5.

As shown in FIG. 6A, in step S101 the controller 41 prepares the first measurement sample by mixing low concentration reagent and blood sample in the first sample preparing part 61. In step S102 the controller 41 supplies low concentration reagent as sheath fluid to the flow cell 71a. In step S103 the controller 41 flows the first measurement sample to the flow cell 71a. In step S104 the controller 41 performs the first measurement by the resistance detector 71.

The resistance detector 71 is configured to obtain a correct measurement result by measuring a measurement sample prepared by mixing fluid of a desired osmotic pressure with a blood sample. The resistance detector 71 also is configured to obtain a correct measurement result by measuring a measurement sample prepared by mixing a conductive fluid with a blood sample. Accordingly, when preparing the first measurement sample, low concentration reagent containing electrolyte and having a desired osmotic pressure is used rather than pure water. For the same reason, low concentration reagent is used rather than pure water as the sheath fluid to flow to the resistance detector 71 in the first measurement.

As shown in FIG. 6B, in step S111 the controller 41 prepares the second measurement sample by mixing hemolytic agent, stain, and blood sample in the second sample preparing part 62. In step S112 the controller 41 supplies pure water as sheath fluid to the flow cell 72a. In step S113 the controller 41 flows the second measurement sample to the flow cell 72a. In step S114 the controller 41 performs the second measurement by the optical detector 72.

As described above, since the second measurement is performed by the optical detector 72, the osmotic pressure and conductivity of the first measurement are not required in the second measurement. Accordingly, pure water is used rather than low concentration reagent as a sheath fluid flowing to the optical detector 72 in the second measurement. Thus, the amount of high concentration reagent consumed can be suppressed since the amount of low concentration reagent is reduced.

As shown in FIG. 6C, in step S121 the controller 41 prepares the third measurement sample by mixing the first measurement sample and hemoglobin hemolytic agent in the first sample preparing part 61. In step S122 the controller 41 supplies pure water to the cell 73a when the cell 73a is not filled with pure water immediately before the third measurement is performed. In step S123 the controller 41 flows the third measurement sample to the cell 73a. In step S124 the controller 41 controls the hemoglobin measuring part 73 to perform the third measurement. When the third measurement concludes, in step S125 the controller 41 discharges the fluid within the cell 73a to the waste chamber 502 and supplies pure water to the cell 73a .

As described above, since the third measurement is performed by the hemoglobin measuring part 73, the osmotic pressure and conductivity of the first measurement are not required in the third measurement. Accordingly, pure water rather than low concentration reagent is used as the fluid to be supplied to the cell 73a in the third measurement. Thus, the amount of high concentration reagent consumed can be suppressed since the amount of low concentration reagent is reduced.

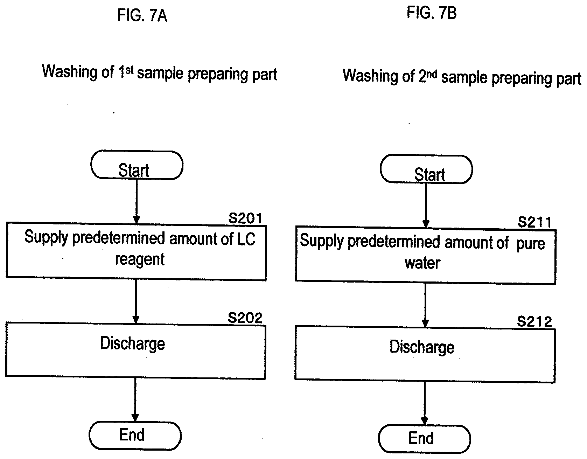

As shown in FIG. 7A, in step S201 the controller 41 supplies a predetermined amount of low concentration reagent to the first sample preparing part 61. The predetermined amount, for example, is greater than the amounts used in the first and third measurement sample which are prepared in case of the first and third measurements. In step S202 the controller 41 discharges the fluid within the first sample preparing part 61 to the waste chamber 501. The washing of the first sample preparing part 61 concludes in this way. The washing of the first sample preparing part 61 is performed each time the first measurement and third measurement conclude for a single blood sample.

When pure water is used to wash the first sample preparing part 61, there is concern that the pure water remaining in the first sample preparing part 61 from the washing may contaminate the first measurement sample when a subsequent first measurement sample is prepared. Accordingly, low concentration reagent rather than pure water is used when washing the first sample preparing part for the same reason as when the first measurement sample is prepared.

As shown in FIG. 7B, in step S211 the controller 41 supplies a predetermined amount of pure water to the second sample preparing part 62. The predetermined amount, for example, is greater than the amount used in the second measurement sample which is prepared in case of the second measurement. In step S212 the controller 41 discharges the fluid within the second sample preparing part 62 to the waste chamber 501. The washing of the second sample preparing part 62 concludes in this way. The washing of the second sample preparing part 62 is performed when the second measurement concludes.

Even though pure water is used to wash the second sample preparing part 62, the pure water remaining from the washing is not a problem when preparing the second measurement sample since the second sample preparing part 62 prepares only the second measurement sample used in the measurement by the optical detector 72. Accordingly, pure water rather than low concentration reagent is used when washing the second sample preparing part 62. Thus, the amount of high concentration reagent consumed can be suppressed since the amount of low concentration reagent is reduced.

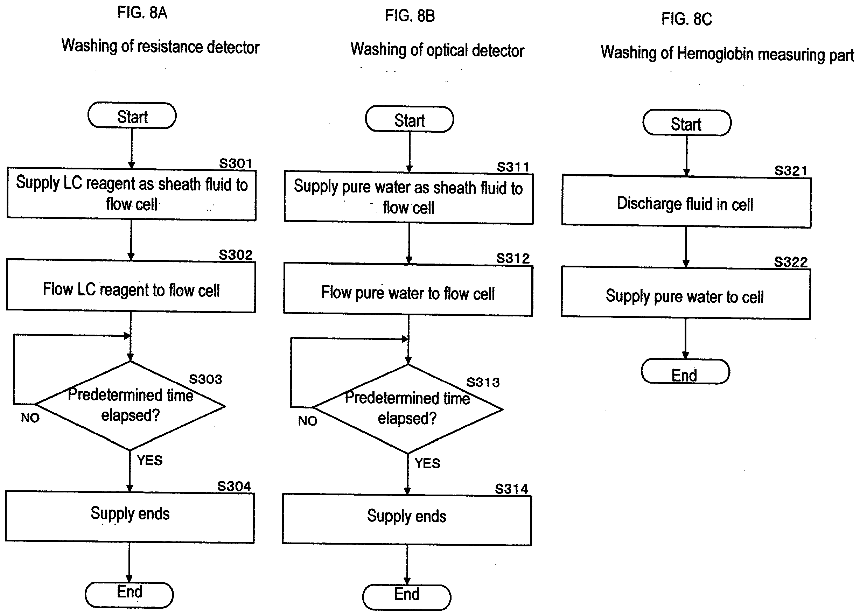

As shown in FIG. 8A, in step S301 the controller 41 supplies low concentration reagent as sheath fluid to the flow cell 71a similar to the case of the first measurement. In step S302 the controller 41 supplies low concentration reagent to the flow cell 71a. In step S303 the controller 41 continues to supply the low concentration reagent started in steps S301 and S302 until a predetermined time, for example, 10 seconds, has elapsed. When the predetermined time has elapsed, in step S304 the controller 41 ends the supply of low concentration reagent started in steps S301 and S302. Washing the resistance detector 71 is performed each time the first measurement concludes.

When pure water is used to wash the resistance detector 71, there is concern that the pure water remaining in the resistance detector 71 from the washing may contaminate the first measurement sample when a subsequent first measurement sample is prepared, such that a correct measurement result will not be obtained. Accordingly, low concentration reagent rather than pure water is used when washing the resistance detector 71.

As shown in FIG. 8B, in step S311 the controller 41 supplies pure water as sheath fluid to the flow cell 72a similar to the case of the second measurement. In step S312 the controller 41 supplies pure water to the flow cell 72a. In step S313 the controller 41 continues to supply the pure water started in steps S311 and S312 until a predetermined time, for example, 10 seconds, has elapsed. When the predetermined time has elapsed, in step S314 the controller 41 ends the supply of pure water started in steps S311 and S312. Washing the optical detector 72 is performed each time the first measurement concludes.

Even though pure water is used to wash the optical detector 72, there is scant effect on the measurement results of the second measurement performed after washing. Accordingly, pure water rather than low concentration reagent is used when washing the optical detector 72. Thus, the amount of high concentration reagent consumed can be suppressed since the amount of low concentration reagent is reduced.

As described above, when the third measurement concludes, the fluid in the cell 73a of the hemoglobin measuring part 73 is discharged and the cell 73a is filled with pure water. In this way there is no particular problem even though a washing operation is not separately performed for the cell 73a. However, a separate washing operation for the cell 73a also may be performed when the third measurement concludes. In this case the process shown in FIG. 8C is executed each time the process concludes for the third measurement shown in FIG. 6C.

As shown in FIG. 8C, in step S321 the controller 41 discharges the fluid in the cell 73a to the waste chamber 502 when the process concludes for the third measurement shown in FIG. 6C. Pure water is supplied to the cell 73a after the third measurement, and the pure water used to wash the cell 73a is discharged in this way. In step S322 the controller 41 again supplies pure water to the cell 73a.

When the hemoglobin measuring part 73 is washed in a separate operation, the contamination of the cell 73a can be cleaned out in this way. Even though pure water is used to wash the hemoglobin measuring part 73 in this way, there is scant effect on the measurement results of the third measurement performed after washing. Accordingly, pure water rather than low concentration reagent is used when washing the hemoglobin measuring part 73. Thus, the amount of high concentration reagent consumed can be suppressed since the amount of low concentration reagent is reduced.

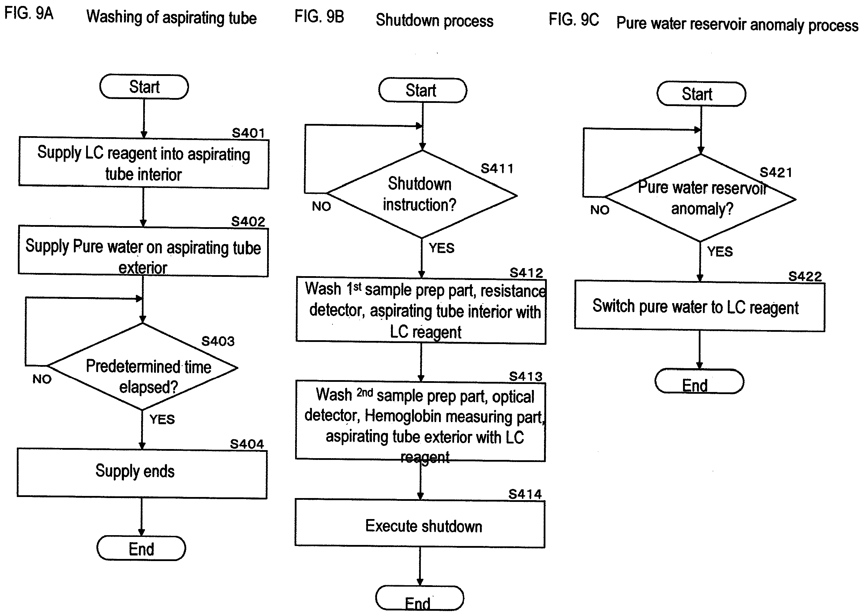

As shown in FIG. 9A, in step S401 the controller 41 supplies low concentration reagent into the interior of the aspirating tube 51. In step S402 the controller 41 supplies pure water to the exterior of the aspirating tube through the washing part 52. The interior of the aspirating tube 51 is washed with low concentration reagent, and the exterior of the aspirating tube 51 is washed with pure water in this way. In step S403 the controller 41 continues to supply the low concentration reagent which began in step S401 and supply the pure water which began in step S402until a predetermined time, for example, 3 seconds, has elapsed. When the predetermined time has elapsed, in step S404 the controller 41 ends the supply started in step S404. Washings of the interior and exterior of the aspirating tube 51 are performed each time aspiration concludes for a single blood sample. Washing of the exterior of the aspirating tube 51 also may be performed every time the aspirating tube 51 is raised for aspiration and discharge.

When pure water is used to wash the interior of the aspirating tube 51, there is concern that the pure water remaining in the aspirating tube 51 from washing may contaminate the first measurement sample during preparation of the first measurement, such that a correct measurement result will not be obtained in the first measurement. Accordingly, low concentration reagent rather than pure water is used when washing the interior of the aspirating tube 51.

When the low concentration reagent is dried, the electrolyte, for example, NaCl, contained in the low concentration reagent precipitates. Therefore, the electrolyte precipitates on the exterior of the aspirating tube 51 when low concentration reagent is used to wash the exterior of the aspirating tube 51. When this electrolyte accumulates on the exterior of the aspirating tube 51, it becomes difficult for the aspirating tube 51 to penetrate the sealed lid of the test tube. It is also assumed that the aspirating tube 51 has difficulty moving vertically through the through-holes 121 and 130 of the washing part 52 shown in FIG. 2A and 2B. Accordingly, pure water rather than low concentration reagent is used when washing the exterior of the aspirating tube 51 so that electrolyte does not accumulate on the exterior of the aspirating tube 51. Electrolyte is prevented from accumulating on the exterior of the aspirating tube 51 in this way. The amount of high concentration reagent consumed also can be suppressed since the amount of low concentration reagent is reduced.

As shown in FIG. 9B, in step S411 the controller 41 determines whether a shutdown instruction has been input for the blood measuring device 10 by the operator operating an operation part which is not shown in the drawing. When a shutdown instruction is input, the controller 41 washes the first measuring part 61, resistance detector 71, and interior of the aspirating tube 51 using low concentration reagent in step S412. Washing the first sample preparing part 61 using low concentration reagent is performed identically to the process shown in FIG. 7A. Washing the resistance detector 71 using low concentration reagent is performed identically to the process shown in FIG. 8A. Washing the interior of the aspirating tube 51 using low concentration reagent is performed identically to the processes of steps S401, S403, and S404 shown in FIG. 9A.

Continuing, in step S413 the controller 41 washes the second sample preparing part 61, optical detector 72, hemoglobin measuring part 73, and exterior of the aspirating tube 51 using low concentration reagent.

Washing the second sample preparing part 62 using low concentration reagent is performed using low concentration reagent rather than pure water in the process shown in FIG. 7B. Specifically, in step S211 the controller 41 supplies a predetermined amount of low concentration reagent from position 11 to the second sample preparing part 62 by opening valve 433.

Washing the optical detector 72 using low concentration reagent is performed using low concentration reagent in place of pure water in the process shown in FIG. 8B. Specifically, in step S311 the controller 41 supplies low concentration reagent from position 13 to the optical detector 72 identically to the sheath fluid by opening valve 430. In step S312 the controller 41 supplies low concentration reagent from position 13 to the optical detector 72 by opening valves 435 and 431.

Washing the hemoglobin measuring part 73 using low concentration reagent is performed by discharging the pure water in the cell 73a and flowing low concentration reagent to the cell 73a. Specifically, the controller 41 sends the pure water filling the cell 73a to the waste chamber 502 by opening valves 423 and 421. Continuing, the controller 41 supplies low concentration reagent from position 12 to the cell 73a of the hemoglobin measuring part 73 by opening valve 420. Then, the controller 41 sends the low concentration reagent in the cell 73a to the waste chamber 502 by opening valves 423 and 421.

Washing the exterior of the aspirating tube 51 using low concentration reagent is performed using low concentration reagent in place of pure water in steps S402 through S404 shown in FIG. 9A. Specifically, in step S402 the controller 41 sends the low concentration reagent from position 13 to the waste chamber 501 through the washing part 52 by opening valves 412 and 411.

After washing is concluded, in step S414 the controller 41 executes shutdown of the blood measuring device 10. There is concern that mold may occur at the washing site supplied with pure water during the washing operation when the blood measuring device 10 is shutdown for a long period. However, the low concentration reagent includes antiseptic agent as mentioned above. Accordingly, when shutdown is performed based on a shutdown operation, and the sites washed using pure water in the washing operation are also washed with low concentration reagent, the formation of mold or the like at these sites can be prevented.

As shown in FIG. 9C, in step S421 the controller 41 determines whether an anomaly has occurred in the pure water reservoir 32. For example, the storage amount of the pure water reservoir 32 is not of a predetermined range even though the preparation controller 31 performs controls to supply the pure water of the pure water tank 24 to the pure water reservoir 32 when the pure water of the pure water tank 24 is decreasing. In this case the controller 41 receives anomaly information indicating an anomaly has occurred from the preparation controller 31, and determines an anomaly exists in the pure water reservoir 32 when the anomaly information is received.

When an anomaly occurs in the pure water reservoir 32, in step S422 the controller 41 switches the pure water to be supplied to the fluid supplying part 42 to low concentration reagent. Specifically, the controller 41 supplies low concentration reagent in place of pure water as a sheath fluid to the optical detector 72. The controller 41 supplies low concentration reagent in place of pure water to the cell 73a of the hemoglobin measuring part 73. The controller 41 supplies low concentration reagent in place of pure water to the second sample preparing part 62, optical detector 72, and the exterior of the aspirating tube 51 when washing. The control of the fluid supplying part 42 by the controller 41 when supplying low concentration reagent in place of pure water is as described in the shutdown process of FIG. 9B.

Thus, the operation of the blood measuring device 10 can continue when an anomaly occurs in the pure water reservoir 32 and the pure water supplied by the fluid supplying part 42 is switched to low concentration reagent.

Note that although low concentration reagent is prepared by diluting high concentration reagent supplied from the high concentration reagent container in the embodiment, the invention is not limited to this arrangement. A low concentration reagent container which contains low concentration reagent also may be directly connected to the blood measuring device, so that blood measurements can be performed using the low concentration reagent supplied from the low concentration reagent container. In this case, a function identical to the embodiment can be realized by supplying pure water from a separately installed pure water unit 20 to the blood measuring device.

## Claims

1. A blood measuring device (10) comprising:
a reagent preparation unit (30) comprising a high concentration reagent reservoir part (33) which stores high concentration reagent, a pure water reservoir part (32) which stores pure water for diluting the high concentration reagent, and a reagent reservoir part (34) which stores low concentration reagent prepared by mixing the pure water and the high concentration reagent; and
a measurement unit (40) comprising a sample preparing part (61, 62) which prepares a measurement sample by mixing a blood sample and the low concentration reagent, and a measuring part (70) which measures red blood cells in the measurement sample,
the blood measuring device (10) being **characterized in that**
the measurement unit (40) further comprises a fluid supplying part (42) which supplies the pure water and the low concentration reagent and is connected to the pure water reservoir part (32) and the reagent reservoir part (34); and that
the blood measuring device (10) further comprises a controller (41) which controls the operation of the fluid supplying part (42), wherein
the controller is configured to control the fluid supplying part (42) to wash one or more parts of the measurement unit (40) least affecting the measurement results using the pure water, and wash other parts of the measurement unit (40) affecting the measurement results using the low concentration reagent, when the measurement unit (40) performs washing operation.

2. The blood measuring device of claim 1, wherein
the measuring part (70) comprises an electrical resistance detecting part (71) and an optical detecting part (72), and
the controller (41) is configured to control the fluid supplying part (42) to wash the optical detecting part (72) using the pure water, and wash the electrical resistance detecting part (71) using the low concentration reagent.

3. The blood measuring device of claim 1 or 2, wherein
the measuring part (70) comprises a hemoglobin measuring part (73) which performs measurements related to hemoglobin; and
the controller (41) is configured to control the fluid supplying part (42) to wash the hemoglobin measuring part (73) using the low concentration reagent.

4. The blood measuring device of any among claims 1 through 3, wherein
the measuring part (70) comprises an electrical resistance detecting part (71) and an optical detecting part (72) ;
the sample preparing part (61, 62) comprises a first sample preparing part (61) which prepares a first measurement sample to be measured by the electrical resistance detecting part (71), and a second sample preparing part (62) which prepares a second measurement sample to be measured by the optical detecting part (72);
the controller (41) is configured to control the fluid supplying part (42) to wash the second sample preparing part (62) using the pure water, and wash the first sample preparing part (61) using the low concentration reagent.

5. The blood measuring device of any among claims 1 through 4, wherein
the measurement unit (40) further comprises an aspirating part (50) which has an aspirating tube (51) for aspirating the blood sample, and a washing part (52) for washing the exterior of the aspirating tube (51);
the controller (41) is configured to control the fluid supplying part (42) to wash the exterior of the aspirating tube (51) using the pure water, and wash the interior of the aspirating tube (51) using the low concentration reagent.

6. The blood measuring device of any among claims 1 through 5, wherein
the measuring part (70) comprises an optical detecting part (72) which has a flow cell (72a); and
the optical detecting part (72) measures the measurement sample flowing with a sheath fluid through the flow cell (72a), and
the pure water is used as the sheath fluid of the flow cell (72a) in the optical detecting part (72).

7. The blood measuring device of any among claims 1 through 6, wherein
the measuring part (70) comprises an electrical resistance detecting part (71) which has a flow cell (71a); and
the electrical resistance detecting part (71) measures the measurement sample flowing with a sheath fluid through the flow cell (71a), and
the low concentration reagent is used as the sheath fluid of the flow cell (71a) in the electrical resistance detecting part (71).

8. The blood measuring device of any among claims 1 through 7, wherein
the controller (41) is configured to control the fluid supplying part (42) to switch the pure water being supplied by the fluid supplying part (42) to the low concentration reagent when an anomaly occurs in the pure water reservoir part (32).

9. The blood measuring device of any among claims 1 through 8, wherein the pure water is RO (reverse osmosis) water.

10. A method of controlling a blood measuring device (10) according to any of claims 1 to 9, the method comprising:
preparing low concentration reagent by mixing a high concentration reagent and pure water;
preparing a measurement sample by mixing a blood sample and the low concentration reagent;
washing one or more parts of the measurement unit (40) least affecting the measurement results using the pure water, and washing other parts of the measurement unit (40) affecting the measurement results using the low concentration reagent.

11. The method of controlling a blood measuring device of claim 10, wherein
the measuring part (70) comprises an electrical resistance detecting part (71) and an optical detecting part (72); and
washing the optical detecting part (72) using the pure water, and washing the electrical resistance detecting part (71) using the low concentration reagent.

12. The method of controlling a blood measuring device of claim 10 or 11, wherein
the measuring part (70) comprises a hemoglobin measuring part (73) which performs measurements related to hemoglobin; and
washing the hemoglobin measuring part (73) using reagent.

13. The method of controlling a blood measuring device of any among claims 10 through 12, wherein
the measuring part (70) comprises an electrical resistance detecting part (71) and an optical detecting part (72) ;
the sample preparing part (61, 62) comprises a first sample preparing part (61) which prepares a first measurement sample to be measured by the electrical resistance detecting part (71), and a second sample preparing part (62) which prepares a second measurement sample to be measured by the optical detecting part (72); and
washing the second sample preparing part (61) using the pure water, and washing the first sample preparing part (62) using the low concentration reagent.

14. The method of controlling a blood measuring device of any among claims 10 through 13, wherein
the measurement unit (40) further comprises an aspirating part (50) which has an aspirating tube (51) for aspirating the blood sample, and a washing part (52) for washing the exterior of the aspirating tube (51); and
washing the exterior of the aspirating tube (51) using the pure water, and washing the interior of the aspirating tube (51) using the low concentration reagent.

15. The method of controlling a blood measuring device of any among claims 10 through 14, wherein
the measuring part (70) comprises an optical detecting part (72) which has a flow cell (72a);
the optical detecting part (72) measures the measurement sample flowing with a sheath fluid through the flow cell (72a), and
the pure water is used as the sheath fluid of the flow cell (72a) in the optical detecting part (72).

## Patentansprüche

1. Blutmessvorrichtung (10), umfassend:
eine Reagenzzubereitungseinheit (30), umfassend einen Hochkonzentrationsreagenzbehälterteil (33), der Hochkonzentrationsreagenz speichert, einen Reinwasserbehälterteil (32), der reines Wasser zum Verdünnen des Hochkonzentrationsreagenzes speichert, und einen Reagenzbehälterteil (34), der Niedrigkonzentrationsreagenz speichert, das durch Mischen des reinen Wassers und des Hochkonzentrationsreagenzes zubereitet wurde; und
eine Messeinheit (40), umfassend einen Probenzubereitungsteil (61, 62), der eine Messprobe durch Mischen einer Blutprobe und des Niedrigkonzentrationsreagenzes zubereitet, und einen Messteil (70), der rote Blutzellen in der Messprobe misst,
wobei die Blutmessvorrichtung (10) **dadurch gekennzeichnet ist, dass**
die Messeinheit (40) weiter einen Fluidzufuhrteil (42) umfasst, der das reine Wasser und das Niedrigkonzentrationsreagenz zuführt und mit dem Reinwasserbehälterteil (32) und dem Reagenzbehälterteil (34) verbunden ist; und dadurch, dass
die Blutmessvorrichtung (10) weiter eine Steuerung (41) umfasst, die die Tätigkeit des Fluidzufuhrteils (42) steuert, wobei
die Steuerung konfiguriert ist, den Fluidzufuhrteil (42) zu steuern, um einen oder mehrere Teile der Messeinheit (40), der oder die die Messergebnisse am wenigsten beeinflussen, unter Verwendung vom reinen Wasser zu waschen und andere Teile der Messeinheit (40), die das Messergebnis beeinflussen, unter Verwendung des Niedrigkonzentrationsreagenzes zu waschen, wenn die Messeinheit (40) eine Waschtätigkeit durchführt.

2. Blutmessvorrichtung nach Anspruch 1, wobei
der Messteil (70) einen Erfassungsteil des elektrischen Widerstands (71) und einen optischen Erfassungsteil (72) umfasst, und
die Steuerung (41) konfiguriert ist, den Fluidzufuhrteil (42) zu steuern, den optischen Erfassungsteil (72) unter Verwendung vom reinen Wasser zu waschen und den Erfassungsteil des elektrischen Widerstands (71) unter Verwendung des Niedrigkonzentrationsreagenzes zu waschen.

3. Blutmessvorrichtung nach Anspruch 1 oder 2, wobei
der Messteil (70) einen Hämoglobinmessteil (73) umfasst, der Messungen bezüglich Hämoglobin durchführt; und
die Steuerung (41) konfiguriert ist, den Fluidzufuhrteil (42) zu steuern, um den Hämoglobinmessteil (73) unter Verwendung des Niedrigkonzentrationsreagenzes zu waschen.

4. Blutmessvorrichtung nach einem der Ansprüche 1 bis 3, wobei
der Messteil (70) einen Erfassungsteil des elektrischen Widerstands (71) und einen optischen Erfassungsteil (72) umfasst;
der Probenzubereitungsteil (61, 62) einen ersten Probenzubereitungsteil (61) umfasst, der eine erste durch den Erfassungsteil des elektrischen Widerstands (71) zu messende Messprobe zubereitet, und einen zweiten Probenzubereitungsteil (62), der eine zweite durch den optischen Erfassungsteil (72) zu messende Messprobe zubereitet;
die Steuerung (41) konfiguriert ist, den Fluidzufuhrteil (42) zu steuern, um den zweiten Probenzubereitungsteil (62) unter Verwendung vom reinen Wasser zu waschen und den ersten Probenzubereitungsteil (61) unter Verwendung des Niedrigkonzentrationsreagenzes zu waschen.

5. Blutmessvorrichtung nach einem der Ansprüche 1 bis 4, wobei
die Messeinheit (40) weiter einen Ansaugteil (50), der ein Ansaugrohr (51) zum Ansaugen der Blutprobe aufweist, und einen Waschteil (52) zum Waschen des Äußeren des Ansaugrohrs (51) umfasst;
die Steuerung (41) konfiguriert ist, den Fluidzufuhrteil (42) zu steuern, um das Äußere des Ansaugrohrs (51) unter Verwendung vom reinen Wasser zu waschen und das Innere des Ansaugrohrs (51) unter Verwendung des Niedrigkonzentrationsreagenzes zu waschen.

6. Blutmessvorrichtung nach einem der Ansprüche 1 bis 5, wobei
der Messteil (70) einen optischen Erfassungsteil (72) umfasst, der eine Strömungszelle (72a) aufweist; und
der optische Erfassungsteil (72) die Messprobe misst, die mit einem Trägerfluid durch die Strömungszelle (72a) fließt, und
das reine Wasser als das Trägerfluid der Strömungszelle (72a) im optischen Erfassungsteil (72) verwendet wird.

7. Blutmessvorrichtung nach einem der Ansprüche 1 bis 6, wobei
der Messteil (70) einen Erfassungsteil des elektrischen Widerstands (71) umfasst, der eine Strömungszelle (71a) aufweist; und
der Erfassungsteil des elektrischen Widerstands (71) die Messprobe misst, die mit einem Trägerfluid durch die Strömungszelle (71a) fließt, und
das Niedrigkonzentrationsreagenz als das Trägerfluid der Strömungszelle (71a) im Erfassungsteil des elektrischen Widerstands (71) verwendet wird.

8. Blutmessvorrichtung nach einem der Ansprüche 1 bis 7, wobei
die Steuerung (41) konfiguriert ist, den Fluidzufuhrteil (42) zu steuern, um das reine Wasser, das vom Fluidzufuhrteil (42) zugeführt wird, zum Niedrigkonzentrationsreagenz umzuschalten, wenn eine Anomalie im Reinwasserbehälterteil (32) auftritt.

9. Blutmessvorrichtung nach einem der Ansprüche 1 bis 8, wobei das reine Wasser RO(Umkehrosmose)-Wasser ist.

10. Verfahren zum Steuern einer Blutmessvorrichtung (10) nach einem der Ansprüche 1 bis 9, wobei das Verfahren umfasst:
Zubereiten von Niedrigkonzentrationsreagenz durch Mischen eines Hochkonzentrationsreagenzes und reinen Wassers;
Zubereiten einer Messprobe durch Mischen einer Blutprobe und des Niedrigkonzentrationsreagenzes;
Waschen eines oder mehrerer Teile der Messeinheit (40), die die Messergebnisse am wenigsten beeinflussen, unter Verwendung vom reinen Wasser, und Waschen anderer Teile der Messeinheit (40), die die Messergebnisse beeinflussen, unter Verwendung des Niedrigkonzentrationsreagenzes.

11. Verfahren zum Steuern einer Blutmessvorrichtung nach Anspruch 10, wobei
der Messteil (70) einen Erfassungsteil des elektrischen Widerstands (71) und einen optischen Erfassungsteil (72) umfasst; und
Waschen des optischen Erfassungsteils (72) unter Verwendung vom reinen Wasser und Waschen des Erfassungsteils des elektrischen Widerstands (71) unter Verwendung des Niedrigkonzentrationsreagenzes.

12. Verfahren zum Steuern einer Blutmessvorrichtung nach Anspruch 10 oder 11, wobei
der Messteil (70) einen Hämoglobinmessteil (73) umfasst, der Messungen bezüglich Hämoglobin durchführt; und
Waschen des Hämoglobinmessteils (73) unter Verwendung von Reagenz.

13. Verfahren zum Steuern einer Blutmessvorrichtung nach einem der Ansprüche 10 bis 12, wobei
der Messteil (70) einen Erfassungsteil des elektrischen Widerstands (71) und einen optischen Erfassungsteil (72) umfasst;
der Probenzubereitungsteil (61, 62) einen ersten Probenzubereitungsteil (61) umfasst, der eine erste Messprobe zubereitet, die vom Erfassungsteil des elektrischen Widerstands (71) zu messen ist, und einen zweiten Probenzubereitungsteil (62), der eine zweite Messprobe zubereitet, die vom optischen Erfassungsteil (72) zu messen ist; und
Waschen des zweiten Probenzubereitungsteils (61) unter Verwendung vom reinen Wasser und Waschen des ersten Probenzubereitungsteils (62) unter Verwendung des Niedrigkonzentrationsreagenzes.

14. Verfahren zum Steuern einer Blutmessvorrichtung nach einem der Ansprüche 10 bis 13, wobei
die Messeinheit (40) weiter einen Ansaugteil (50) umfasst, der ein Ansaugrohr (51) zum Ansaugen der Blutprobe aufweist, und einen Waschteil (52) zum Waschen des Äußeren des Ansaugrohrs (51); und
Waschen des Äußeren des Ansaugrohrs (51) unter Verwendung vom reinen Wasser und Waschen des Inneren des Ansaugrohrs (51) unter Verwendung des Niedrigkonzentrationsreagenzes.

15. Verfahren zum Steuern einer Blutmessvorrichtung nach einem der Ansprüche 10 bis 14, wobei
der Messteil (70) einen optischen Erfassungsteil (72) umfasst, der eine Strömungszelle (72a) aufweist;
der optische Erfassungsteil (72) die Messprobe misst, die mit einem Trägerfluid durch die Strömungszelle (72a) fließt, und
das reine Wasser als das Trägerfluid der Strömungszelle (72a) im optischen Erfassungsteil (72) verwendet wird.

## Revendications

1. Dispositif de mesure de sang (10) comprenant :
une unité de préparation de réactif (30) comprenant une partie réservoir de réactif à concentration élevée (33) qui stocke un réactif à concentration élevée, une partie réservoir d'eau pure (32) qui stocke de l'eau pure pour diluer le réactif à concentration élevée, et une partie réservoir de réactif (34) qui stocke du réactif à concentration basse préparé en mélangeant l'eau pure et le réactif à concentration élevée ; et
une unité de mesure (40) comprenant une partie de préparation d'échantillon (61, 62) qui prépare un échantillon de mesure en mélangeant un échantillon de sang et le réactif à concentration basse, et une partie de mesure (70) qui mesure des globules rouges dans l'échantillon de mesure,
le dispositif de mesure de sang (10) étant **caractérisé en ce que**
l'unité de mesure (40) comprend en outre une partie de fourniture de fluide (42) qui fournit l'eau pure et le réactif à concentration basse et est reliée à la partie réservoir d'eau pure (32) et à la partie réservoir de réactif (34) ; et **en ce que**
le dispositif de mesure de sang (10) comprend en outre une unité de commande (41) qui commande le fonctionnement de la partie de fourniture de fluide (42), dans lequel
l'unité de commande est configurée pour commander la partie de fourniture de fluide (42) pour laver une ou plusieurs parties de l'unité de mesure (40) affectant le moins les résultats de mesure en utilisant l'eau pure et pour laver d'autres parties de l'unité de mesure (40) affectant les résultats de mesure en utilisant le réactif à concentration basse, lorsque l'unité de mesure (40) effectue une opération de lavage.

2. Dispositif de mesure de sang selon la revendication 1, dans lequel
la partie de mesure (70) comprend une partie de détection de résistance électrique (71) et une partie de détection optique (72), et
l'unité de commande (41) est configurée pour commander la partie de fourniture de fluide (42) pour laver la partie de détection optique (72) en utilisant l'eau pure, et laver la partie de détection de résistance électrique (71) en utilisant le réactif à concentration basse.

3. Dispositif de mesure de sang selon la revendication 1 ou 2, dans lequel
la partie de mesure (70) comprend en outre une partie de mesure d'hémoglobine (73) qui effectue des mesures liées à l'hémoglobine ; et
l'unité de commande (41) est configurée pour commander la partie de fourniture de fluide (42) pour laver la partie de mesure d'hémoglobine (73) en utilisant le réactif à concentration basse.

4. Dispositif de mesure de sang selon l'une quelconque des revendications 1 à 3, dans lequel
la partie de mesure (70) comprend une partie de détection de résistance électrique (71) et une partie de détection optique (72) ;
la partie de préparation d'échantillon (61, 62) comprend une première partie de préparation d'échantillon (61) qui prépare un premier échantillon de mesure à mesurer par la partie de détection de résistance électrique (71) et une seconde partie de préparation d'échantillon (62) qui prépare un second échantillon de mesure à mesurer par la partie de détection optique (72) ;
l'unité de commande (41) est configurée pour commander la partie de fourniture de fluide (42) pour laver la seconde partie de préparation d'échantillon (62) en utilisant l'eau pure, et laver la première partie de préparation d'échantillon (61) en utilisant le réactif à concentration basse.

5. Dispositif de mesure de sang selon l'une quelconque des revendications 1 à 4, dans lequel
l'unité de mesure (40) comprend en outre une partie d'aspiration (50) qui a un tube d'aspiration (51) pour aspirer l'échantillon de sang, et une partie de lavage (52) pour laver l'extérieur du tube d'aspiration (51);
l'unité de commande (41) est configurée pour commander la partie de fourniture de fluide (42) pour laver l'extérieur du tube d'aspiration (51) en utilisant l'eau pure, et laver l'intérieur du tube d'aspiration (51) en utilisant le réactif à concentration basse.

6. Dispositif de mesure de sang selon l'une quelconque des revendications 1 à 5, dans lequel
la partie de mesure (70) comprend une partie de détection optique (72) qui a une cellule de flux (72a) ; et
la partie de détection optique (72) mesure l'échantillon de mesure s'écoulant avec un fluide de gaine à travers la cellule de flux (72a), et
l'eau pure est utilisée en tant que fluide de gaine de la cellule de flux (72a) dans la partie de détection optique (72).

7. Dispositif de mesure de sang selon l'une quelconque des revendications 1 à 6, dans lequel
la partie de mesure (70) comprend une partie de détection de résistance électrique (71) qui a une cellule de flux (71a) ; et
la partie de détection de résistance électrique (71) mesure l'échantillon de mesure s'écoulant avec un fluide de gaine à travers la cellule de flux (71a), et
le réactif à concentration basse est utilisé en tant que fluide de gaine de la cellule de flux (71a) dans la partie de détection de résistance électrique (71).

8. Dispositif de mesure de sang selon l'une quelconque des revendications 1 à 7, dans lequel
l'unité de commande(41) est configurée pour commander la partie de fourniture de fluide (42) pour passer l'eau pure qui est fournie par la partie de fourniture de fluide (42) au réactif à concentration basse quand une anomalie se produit dans la partie réservoir d'eau pure (32).

9. Dispositif de mesure de sang selon l'une quelconque des revendications 1 à 8, dans lequel l'eau pure est de l'eau obtenue par RO (osmose inverse).

10. Procédé de commande d'un dispositif de mesure de sang (10) selon l'une quelconque des revendications 1 à 9, le procédé comprenant :
la préparation du réactif à concentration basse en mélangeant un réactif à concentration élevée et de l'eau pure ;
la préparation d'unéchantillon de mesure en mélangeant un échantillon de sang et le réactif à concentration basse ;
le lavage d'une ou plusieurs parties de l'unité de mesure (40) affectant le moins les résultats de mesure en utilisant l'eau pure, et le lavage d'autres parties de l'unité de mesure (40) affectant les résultats de mesure en utilisant le réactif à concentration basse.

11. Procédé de commande d'un dispositif de mesure de sang selon la revendication 10, dans lequel
la partie de mesure (70) comprend une partie de détection de résistance électrique (71) et une partie de détection optique (72) ; et
le lavage de la partie de détection optique (72) en utilisant l'eau pure, et
le lavage de la partie de détection de résistance électrique (71) en utilisant le réactif à concentration basse.

12. Procédé de commande d'un dispositif de mesure de sang selon la revendication 10 ou 11, dans lequel
la partie de mesure (70) comprend une partie de mesure d'hémoglobine (73) qui effectue des mesures liées à l'hémoglobine ; et
le lavage de la partie de mesure d'hémoglobine (73) en utilisant du réactif.

13. Procédé de commande d'un dispositif de mesure de sang selon l'une quelconque des revendications 10 à 12, dans lequel
la partie de mesure (70() comprend une partie de détection de résistance électrique (71) et une partie de détection optique (72);
la partie de préparation d'échantillon (61, 62) comprend une première partie de préparation d'échantillon (61) qui prépare un premier échantillon de mesure à mesurer par la partie de détection de résistance électrique (71) et une seconde partie de préparation d'échantillon (62) qui prépare un second échantillon de mesure à mesurer par la partie de détection optique (72); et
le lavage de la seconde partie de préparation d'échantillon (61) en utilisant l'eau pure, et le lavage de la première partie de préparation d'échantillon (62) en utilisant le réactif à concentration basse.

14. Procédé de commande d'un dispositif de mesure de sang selon l'une quelconque des revendications 10 à 13, dans lequel
l'unité de mesure (40) comprend en outre une partie d'aspiration (50) qui a un tube d'aspiration (51) pour aspirer l'échantillon de sang, et une partie de lavage (52) pour laver l'extérieur du tube d'aspiration (51) ; et
le lavage de l'extérieur du tube d'aspiration (51) en utilisant l'eau pure et le lavage de l'intérieur du tube d'aspiration (51) en utilisant le réactif à concentration basse.

15. Procédé de commande d'un dispositif de mesure de sang selon l'une quelconque des revendications 10 à 14, dans lequel
la partie de mesure (70) comprend une partie de détection optique (72) qui a une cellule de flux (72a) ;
la partie de détection optique (72) mesure l'échantillon de mesure s'écoulant avec un fluide de gaine à travers la cellule de flux (72a), et
l'eau pure est utilisée en tant que fluide de gaine de la cellule de flux (72a) dans la partie de détection optique (72).
